# EUROPÄISCHE PATENTSCHRIFT

(11) **EP 1 400 691 B1**
(45) Veröffentlichungstag und Bekanntmachung des Hinweises auf die Patenterteilung: **18.07.2007**
(21) Anmeldenummer: 03405652.3
(22) Anmeldetag: 08.09.2003
(51) Int. Cl.: F04B 43/12

(54) **Schlauchkassette für eine peristaltische Pumpe**
Tube cassette for peristaltic pump
Cassette à tube de pompe péristaltique

(30) Priorität: 23.09.2002 DE 10244090
(43) Veröffentlichungstag der Anmeldung: 24.03.2004
(73) Patentinhaber: Ismatec SA, Laboratoriumstechnik, 8152 Glattbrugg (CH)
(72) Erfinder: Saxer, Daniel, 8048 Zürich (CH); Fässler, René, 8049 Zürich (CH); Michels, Stephan, 8474 Dinhard (CH)
(74) Vertreter: Rottmann, Maximilian

(56) Entgegenhaltungen:
- EP-A- 1 108 891
- US-A- 3 597 124
- US-A- 4 184 815
- US-A- 4 187 057
- US-A- 4 673 334
- US-A- 4 886 431

## Beschreibung

Die Erfindung betrifft eine Schlauchkassette für eine peristaltische Pumpe gemäss dem Oberbegriff des Anspruchs 1.

Mittels einer derartigen Schlauchkassette soll erreicht werden, dass der das zu fördernde Medium aufnehmende Schlauchabschnitt einfach und schnell an der Pumpe befestigt werden kann. Da derartige Schlauchkassetten grundsätzlich bekannt sind, wird nachfolgend nur auf die im Zusammenhang mit der vorliegenden Erfindung relevanten oder in Zusammenhang stehenden Merkmale eingegangen.

Um den in der Schlauchkassette aufzunehmenden Schlauch bzw. Schlauchabschnitt schnell, einfach und mit einer definierten Vorspannung darin fixieren zu können, sind Ausführungsformen von Schläuchen bekannt, die mit zwei Stoppern versehen sind. Der Abstand zwischen diesen beiden Stoppern ist weitgehend normiert und beträgt beispielsweise ca. 140mm. Das Fixieren eines Schlauchabschnitts in den bisherigen, nach dem Stand der Technik ausgebildeten Schlauchkassetten bereitet im allgemeinen keine Probleme, da die Schlauchkassetten physisch so gross sind, dass der Schlauchabschnitt mit dem einen Stopper am einen Ende und mit dem anderen Stopper am anderen Ende der Schlauchkassette fixiert werden kann.

Aus der EP 1 137 886 ist eine Rollenpumpe zur peristaltischen Förderung von Medien bekannt, die mit einer Schlauchkassette versehen ist. Der Schlauch ist in Längsrichtung durch die Schlauchkassette hindurchgeführt, d.h. er verlässt die Schlauchkassette auf der dem Eingang gegenüberliegenden Seite. Die Schlauchkassette wird mittels eines verschliessbaren Deckels an der Rollenpumpe fixiert. Wie aus der Darstellung gemäss Fig. 1 dieser Schrift ersichtlich ist, besitzt eine derartige Schlauchkassette eine relativ grosse Länge und benötigt daher viel Platz.

Aus der EP 1 108 891 ist ebenfalls eine Schlauchkassette für eine peristaltische Pumpe bekannt. Das Kassettengehäuse der Schlauchkassette ist mit einer Ausnehmung zum Eingriff eines Rollenrades der peristaltischen Pumpe versehen. Diese Ausnehmung wird auf der einen Seite von einem U-förmigen Vorderteil begrenzt, an dessen Innenseite der Schlauch anliegt. Der Schlauch selber ist am einen Ende mit einem als Verbindungselement bezeichneten ersten Stopper versehen, der im Kassettengehäuse mittels Anschlagflächen fixiert ist. Am anderen Ende des Schlauchs ist ein weiteres Verbindungselement vorgesehen, welches in Längsrichtung des Schlauchs zwischen einer Montageposition und einer Betriebsposition verschiebbar und in der letzteren auch fixierbar ist.

In der US 3,597,124 ist eine peristaltische Pumpe beschrieben, die u.a. mit einer als "tubing support means" bezeichneten Schlauchabstützung versehen ist. Die Schlauchabstützung ist mittels eines Bolzens drehbar am Gehäuse der Pumpe abgestützt und kann zwischen einer Wirk- und einer Ruhestellung verschwenkt werden. Um die Schlauchabstützung in der Wirkstellung zu blockieren, ist ein drehbarer Knopf vorgesehen, der die Form eines Nockens aufweist. Die verschwenkbare Lagerung der Schlauchabstützung soll sicherstellen, dass der Schlauch in der Ruhestellung einfach angebracht bzw. entfernt werden kann, während in der Wirkstellung der Schlauch durch die Pumprollen abquetschbar sein soll. Zum Fixieren des Pumpschaluchs ist die Schlauchabstützung mit Haltevorsprüngen, retaining lugs genannt, versehen. Am Pumschlauch angebrachte Stopper, welche dem Positionieren und Fixieren des Pumpschauchs dienen, sind keine vorgesehen.

In der US 4,184,815 ist ebenfalls eine peristaltische Pumpe offenbart, die mit einer "Base" genannten Schlauchaufnahme versehen ist, die mit einer Einlass- und einer Auslassstation versehen ist. Vor der jeweiligen Station ist ein Schenkel angeordnet, der eine Nut begrenzt, die zur Aufnahme eines am Pumschlauch angebrachten Stoppers vorgesehen ist. Die Schlauchaufnahme ist mit einem U-förmig gestalteten Wandteil zur Aufnahme des Pumpschlauchs versehen. Im Zentrum der Schlauchaufnahme ist der Rotor angeordnet, der mit zwei Pumprollen versehen ist, die an Federarmen abgestützt sind. Das Fixieren des Pumpschlauch in der Schlauchaufnahme ist relativ aufwändig, zumal dazu auch noch der Rotor verdreht werden muss.

Schliesslich ist in der US 4,886,431 eine peristaltische Pumpe beschrieben, die mit einer Mehrzahl von austauschbaren Kassetten versehen ist. Die jeweilige Kassette besteht aus einem U-förmig ausgebildeten Rahmen, bestehend aus zwei Seitenteilen und einem Oberteil. Im Rahmen ist ein Schlauchbett aufgenommen, das mittels einem Verstellmechanismus entlang der beiden Seitenteile des Rahmens verschiebbar ist. Der Pumpschlauch wird in Längsrichtung durch die Schlauchkassette hindurchgeführt, so dass er auf der dem Eingang gegenüberliegenden Seite aus der Schlauchkassette herausgeführt ist. Eine solche Kassette weist u.a. die bereist eingangs erwähnten Nachteile auf, indem sie gross ist und viel Platz benötigt.

Da auch auf dem Gebiet von peristaltischen Pumpen der Trend immer mehr hin zu kompakten Geräten geht, besteht natürlich grundsätzlich auch der Wunsch nach kompakten Schlauchkassetten.

Der Erfindung liegt deshalb die Aufgabe zugrunde, eine gemäss dem Oberbegriff des Anspruchs 1 ausgebildete Schlauchkassette massgeblich zu verkleinern, wobei darin trotzdem Schlauchabschnitte fixierbar sein sollen, deren Stopper einen insbesondere für grössere Schlauchkassetten vorgegebenen Mindestabstand aufweisen, und wobei die Schlauchkassette derart gestaltet sein soll, dass sie schnell und einfach an der Pumpe fixierbar ist.

Diese Aufgabe wird mit einer Schlauchkassette gelöst, die mit den im Kennzeichen des Anspruchs 1 angeführten Merkmalen versehen ist.

Vorteilhafte Weiterbildungen der Schlauchkassette sind in den abhängigen Ansprüchen 2 bis 9 definiert.

Nachfolgend wird ein bevorzugtes Ausführungsbeispiel der Schlauchkassette anhand von Zeichnungen näher erläutert. In diesen Zeichnungen zeigt:
- Fig. 1: die Schlauchkassette in einer Seitenansicht;
- Fig. 2: die Schlauchkassette gemäss Fig. 1 in einer Ansicht von oben;
- Fig. 3: die Schlauchkassette gemäss Fig. 1 in einer Ansicht von hinten, und
- Fig. 4: eine perspektivische Ansicht der Schlauchkassette zusammen mit einem darin fixierten Schlauchabschnitt.

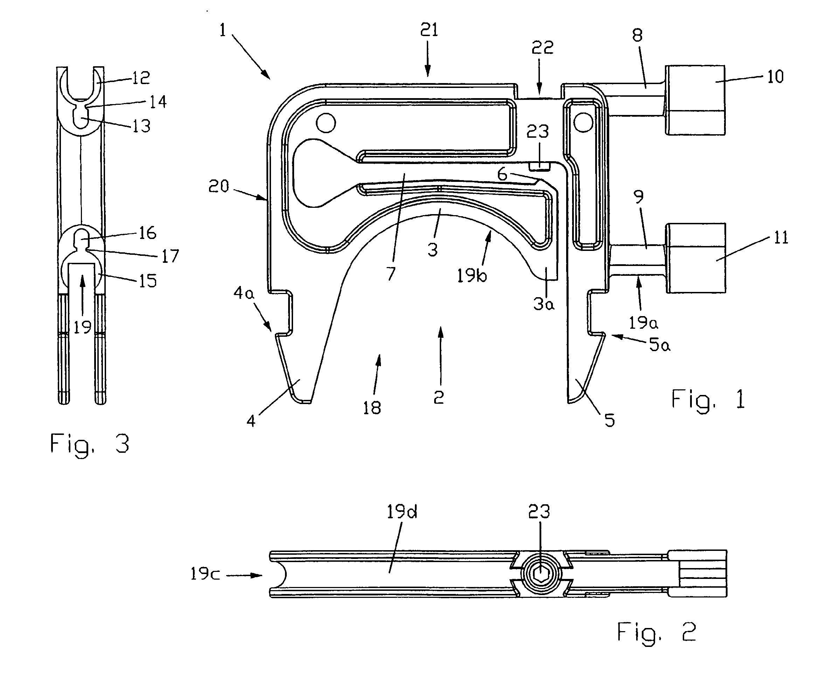

Anhand der Figuren 1 bis 3 wird der grundsätzliche Aufbau der Schlauchkassette näher erläutert. Diese weist ein Kassettengehäuse 1 auf, das mit einer seitlichen Ausnehmung versehen ist. Diese Ausnehmung 2 wird nach innen von einem kreissegmentförmig gestalteten Auslegerarm 3 begrenzt. Zwischen dem Auslegerarm 3 und dem oberen Teil des Kassettengehäuses 1 ist ein Schlitz 7 in das Gehäuse eingelassen. Der Auslegerarm 3 ist nur auf der einen Seite am Kassettengehäuse 1 fixiert, wodurch dieser in radialer Richtung, insbesondere zu seinem Ende 3a hin, federelastisch nachgiebig ist. Die Unterseite des Kassettengehäuses 1 wird durch je zwei Schenkelpaare 4, 5 gebildet, wobei jeder Schenkel auf der Vorderseite mit je einer Rastnase 4a, 5a versehen ist. Die beiden Schenkelpaare 4, 5 begrenzen die Ausnehmung 2 auf beiden Seiten. Indem ein Schlitz 7 im Kassettengehäuse 1 vorgesehen ist und der Auslegerarm 3 nur auf der einen Seite am Kassettengehäuse 1 fixiert ist, sind auch die beiden Schenkelpaare 4, 5 federelastisch gegeneinander bewegbar und ermöglichen ein schnelles Fixieren des Kassettengehäuses 1 an der Pumpe (nicht dargestellt) oder an entsprechenden Fortsätzen derselben. Auf der in Blickrichtung rechten Seite des Kassettengehäuses 1 sind zwei Tragarme 8, 9 angeformt, welche endseitig mit im Durchmesser vergrösserten Aufnahmen 10, 11 versehen sind. In die jeweilige Aufnahme 10, 11 ist je eine grössere und eine kleinere Aussparung 12, 13; 15, 16 zur Aufnahme von an dem jeweiligen Schlauchabschnitt angeformten, zweiteiligen Stoppern eingelassen. Der an dem Kassettengehäuse 1 zu fixierende Schlauchabschnitt mit den daran fixierten Stoppern ist aus den Darstellungen gemäss den Figuren 1 bis 3 nicht ersichtlich. Aus der Ansicht gemäss Fig. 2 geht hervor, dass die jeweils grössere dieser Aussparungen 12, 15 im wesentlichen rund ausgebildet ist, während die jeweils kleinere Aussparung 13, 16 eine im wesentlichen ovale Form aufweist. Zwischen den beiden Aussparungen 12, 13 bzw. 15, 16 ist jeweils eine Einschnürung 14, 17 zum klemmenden Fixieren des jeweiligen Stoppers vorgesehen.

Eine im wesentlichen auf der Aussenseite des Kassettengehäuses 1 umlaufende Nut ist grundsätzlich mit dem Bezugszeichen 19 versehen, wobei die einzelnen Nutabschnitte mit einem zusätzlichen Buchstaben (a, b, c, d) versehen sind. Diese Nut 19 dient der Aufnahme und Führung eines Schlauchabschnitts, wie nachfolgend noch näher erläutert wird. Der erste Nutabschnitt 19a verläuft von der Aufnahme 11 über die Unterseite des unteren Tragarms 9 hinweg und führt durch das rechte Schenkelpaar 5 hindurch. Die in die Unterseite des Auslegerarms 3 eingelassene Nut bzw. der Nutabschnitt trägt das Bezugszeichen 19b. Die Nut 19 erstreckt sich danach durch das linke Schenkelpaar 4 hindurch und führt auf der den beiden Tragarmen 8, 9 gegenüberliegenden Aussenseite -Stirnseite 20- des Kassettengehäuses 1 entlang eines mit 19c bezeichneten Nutabschitts auf die Oberseite 21 des Kassettengehäuses 1, von wo sich ein Nutabschnitt 19d entlang der Oberseite des Kassettengehäuses 1 über den oberen Tragarm 8 hinweg durch die Aufnahme 10 hindurch erstreckt. Vereinfacht ausgedrückt erstreckt sich die Nut 19 vom unteren Tragarm 9 entlang von drei Aussenseiten 18, 20, 21 des Kassettengehäuses 1 bis zum oberen Tragarm 8.

Das Kassettengehäuse 1 ist zudem mit einer vertikal verlaufenden Gewindebohrung 22 zur Aufnahme einer Inbusschraube 23 versehen. Mittels dieser an einem Nocken 6 des Auslegerarms 3 angreifenden Inbusschraube 23 kann die Auslenkung des Auslegerarms 3 verändert werden, wodurch sich der Anpressdruck des jeweiligen Rollenrades der peristaltischen Pumpe in Bezug auf den jeweiligen Schlauchabschnitt 25 verändert, wenn die Schlauchkassette an der Pumpe fixiert ist.

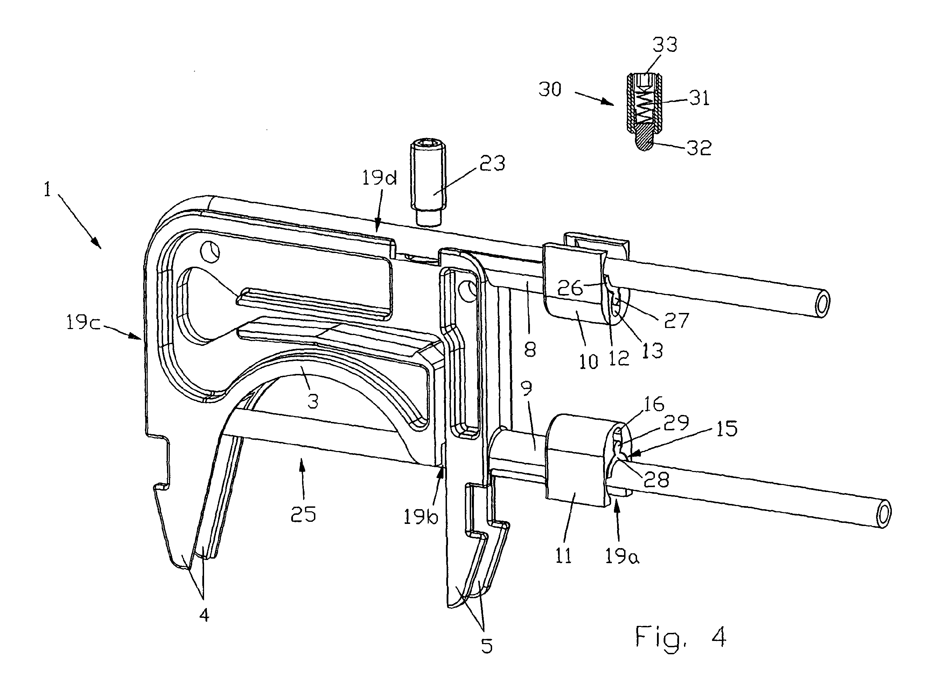

Fig. 4 zeigt eine perspektivische Ansicht der Schlauchkassette zusammen mit einem darin fixierten Schlauchabschnitt 25. In dem hier gezeigten Zustand ist der Schlauchabschnitt 25 mittels zwei Stoppern 26, 28 in der Aufnahme 10, 11 des jeweiligen Tragarms 8, 9 fixiert. Der im unbelasteten Zustand dargestellte Schlauchabschnitt 25 verläuft in diesem Fall gerade zwischen den beiden Schenkelpaaren 4, 5. Es versteht sich, dass der Schlauchabschnitt 25 von der jeweiligen Rolle des Rollenrads der peristaltischen Pumpe in die als Schlauchbett dienende Nut 19b des Auslegerarms 3 hineingedrückt wird, wenn die Schlauchkassette an der Pumpe fixiert ist. Trotz der Kompaktheit der Schlauchkassette weist das Kassettengehäuse 1 eine Nut auf, deren Länge auf die weitverbreiteten 2-Stopperschläuche mit einem relativ weiten Stopperabstand abgestimmt ist, so dass die genannten standardisierten Stopperschläuche erstmalig auf einer derart kompakten Schlauchkassette verwendet werden können.

Aus der Darstellung gemäss Fig. 4 ist zudem ersichtlich, dass der jeweilige Stopper 26, 28 einen radialen Fortsatz 27, 29 aufweist, der in der entsprechenden Ausnehmung 13, 16 der jeweilige Aufnahme 10, 11 aufgenommen ist und sicherstellt, dass sich der im Kassettengehäuse 1 fixierte Schlauchabschnitt 25 in Relation zum Kassettengehäuse 1 nicht verdreht. Im weiteren ist ersichtlich, dass der Schlauchabschnitt 25 von derselben Seite in das Kassettengehäuse 1 hinein- und auch wieder hinausgeführt wird. Anstelle der erwähnten Inbusschraube 23 kann beispielsweise auch ein federnder Stift 30 vorgesehen werden. Der in einem Längsschnitt dargestellte Stift 30 weist einen mittels einer Druckfeder 31 abgestützten Vorderteil 32 auf, der bei in das Kassettengehäuse 1 eingeschraubtem Stift 30 am Auslegerarm 3 zur Anlage kommt und diesen federnd abstützt. Die Vorspannung der Druckfeder 31 kann mittels eines Gewindebolzens 33 verändert werden. Bei der Verwendung eines solchen Stifts 30 wird der Auslegerarm 3 somit gefedert gegen den jeweiligen Schlauchabschnitt gedrückt.

Bei der erfindungsgemässen Schlauchkassette kann der Schlauchabschnitt 25 auf einfachste Weise darin fixiert werden, indem zuerst der eine Stopper 28 in die Ausnehmung 15 der einen Aufnahme 11 eingeführt wird und der Schlauchabschnitt 25 danach um das Kassettengehäuse 1 herumgeführt wird und durch Zugbelastung soweit gestreckt wird, bis der zweite Stopper 26 in die Ausnehmung 12 der anderen Aufnahme 10 eingeführt werden kann. Sobald der Schlauchabschnitt 25 entlastet wird, zieht dieser den Stopper 26 in die genannte Ausnehmung 12 der Aufnahme 10 hinein. Die mit dem Schlauchabschnitt 25 versehene Schlauchkassette kann sehr schnell und einfach an der Pumpe oder entsprechenden Fortsätzen derselben fixiert werden. Insbesondere kann das Rollenrad der peristaltischen Pumpe über die Ausnehmung lateral an die Schlauchkassette herangeführt werden und die Schlauchkassette braucht nicht wie bei den meisten der herkömmlichen Schlauchkassetten über eine zentrale Öffnung auf das Rollenrad der peristaltischen Pumpe aufgeschoben zu werden. Nebst der Tatsache, dass eine derartige Schlauchkassette sehr kompakt ausgebildet ist, ist ein weiterer Vorteil darin zu sehen, dass mehrere Schlauchkassetten parallel und unabhängig voneinander an der mit entsprechenden Fortsätzen versehenen Pumpe fixierbar und auch wieder entfernbar sind.

Indem die beiden Tragarme 8, 9 auf derselben Seite des Kassettengehäuses 1 angeordnet sind, wird auch der Schlauchabschnitt 25 von derselben Seite in das Kassettengehäuse 1 hinein- und auch wieder hinausgeführt. Dadurch wird der Platzbedarf der gesamten Schlauchkassette weiter verringert.

## Patentansprüche

1. Schlauchkassette für eine peristaltische Pumpe, mit einem mit einer seitlichen Ausnehmung (2) versehenen Kassettengehäuse (1), in welche Ausnehmung (2) ein Rollenrad der peristaltischen Pumpe einzugreifen bestimmt ist, wobei die Ausnehmung (2) zumindest teilweise von einem kreissegmentförmigen Element (3) begrenzt wird, welches zumindest teilweise mit einer Nut (19) zur Aufnahme eines Schlauchabschnitts (25) versehen ist, und wobei das Kassettengehäuse (1) mit Mitteln (4, 4a, 5, 5a) zum Fixieren an der Pumpe versehen ist, **dadurch gekennzeichnet, dass** die Nut (19) zur Aufnahme des Schlauchabschnitts (25) über die dem kreissegmentförmigen Element (3) gegenüberliegende Aussenseite (21) des Kassettengehäuses (1) hinaus derart weitergeführt ist, dass der in der Nut (19) aufzunehmende Schlauchabschnitt (25) von derselben Seite her in das Kassettengehäuse (1) hinein- und auch wieder hinausführbar ist, und dass am Kassettengehäuse (1) zwei Tragarme (8, 9) angeordnet sind, welche endseitig mit je einer Aufnahme (10, 11) zum Aufnehmen von am Schlauchabschnitt (25) fixierten Stoppern (26, 28) versehen sind.

2. Schlauchkassette nach Anspruch 1, wobei zumindest zwei Aussenseiten (20, 21) des Kassettengehäuses (1) mit einem Nutabschnitt (19b, 19c) zur Bildung der den Schlauchabschnitt (25) aufnehmenden Nut (19) versehen sind.

3. Schlauchkassette nach Anspruch 1 oder 2, wobei drei Aussenseiten (18, 20, 21) des Kassettengehäuses (1) mit einem Nutabschnitt (19a, 19b, 19c) zur Bildung der den Schlauchabschnitt (25) aufnehmenden Nut (19) versehen sind, und dass an der vierten Aussenseite das Kassettengehäuse (1) zwei Tragarme (8, 9) angeordnet sind.

4. Schlauchkassette nach einem der vorhergehenden Ansprüche, wobei die jeweilige Aufnahme (10, 11) mit zwei Ausnehmungen (12, 13; 15, 16) zur Aufnahme von je einem zweiteiligen Stopper (26, 28) versehen ist.

5. Schlauchkassette nach Anspruch 4, wobei zwischen den beiden Ausnehmungen (12, 13; 15, 16) jeweils eine Einschnürung (14, 17) zum klemmenden Fixieren des jeweiligen Stoppers (26, 28) vorgesehen ist.

6. Schlauchkassette nach einem der vorhergehenden Ansprüche, wobei das kreissegmentförmige Element als federnd am Kassettengehäuse (1) abgestützter Auslegerarm (3) ausgebildet ist.

7. Schlauchkassette nach Anspruch 6, wobei Mittel (23) zum Verändern der Auslenkung des Auslegerarms (3) vorgesehen sind.

8. Schlauchkassette nach einem der vorhergehenden Ansprüche, wobei als Mittel zum Fixieren des Kassettengehäuses (1) an der Pumpe zwei Schenkelpaare (4, 5) vorgesehen sind, wobei an jedem Schenkelpaar (4, 5) zumindest eine Rastnase (4a, 5a) angeordnet ist.

9. Schlauchkassette nach Anspruch 8, wobei die Nut (19) durch die beiden Schenkelpaare (4, 5) hindurch verläuft und die seitliche Ausnehmung (2) im Kassettengehäuse (1) durch die beiden Schenkelpaare (4, 5) auf beiden Seiten begrenzt wird.

## Claims

1. Tube cassette for a peristaltic pump, having a cassette housing (1) which is provided with a lateral recess (2), into which recess (2) a roller wheel of the peristaltic pump is intended to engage, the recess (2) being delimited at least partially by a circular segment-shaped element (3) which is provided at least partially with a groove (19) for accommodating a tube section (25), and the cassette housing (1) being provided with means (4, 4a, 5, 5a) for fixing to the pump, **characterized in that** the groove (19) for accommodating the tube section (25) is guided further beyond that outer side (21) of the cassette housing (1) which lies opposite the circular segment-shaped element (3), in such a way that the tube section (25) which is to be accommodated in the groove (19) can be guided into the cassette housing (1) and also out of it again from the same side, and **in that** two carrying arms (8, 9) which are provided at the end with in each case one receptacle (10, 11) for receiving stoppers (26, 28) which are fixed to the tube section (25) are arranged on the cassette housing (1).

2. Tube cassette according to Claim 1, at least two outer sides (20, 21) of the cassette housing (1) being provided with a groove section (19b, 19c) for forming the groove (19) which accommodates the tube section (25).

3. Tube cassette according to Claim 1 or 2, three outer sides (18, 20, 21) of the cassette housing (1) being provided with a groove section (19a, 19b, 19c) for forming the groove (19) which accommodates the tube section (25), and two carrying arms (8, 9) being arranged on the fourth outer side of the cassette housing (1).

4. Tube cassette according to one of the preceding claims, the respective receptacle (10, 11) being provided with two recesses (12, 13; 15, 16) for receiving in each case one two-part stopper (26, 28).

5. Tube cassette according to Claim 4, in each case one constriction (14, 17) being provided between the two recesses (12, 13; 15, 16), for fixing the respective stopper (26, 28) in a clamped manner.

6. Tube cassette according to one of the preceding claims, the circular segment-shaped element being configured as an outrigger arm (3) which is supported in a sprung manner on the cassette housing (1).

7. Tube cassette according to Claim 6, means (23) for changing the deflection of the outrigger arm (3) being provided.

8. Tube cassette according to one of the preceding claims, two pairs (4, 5) of limbs being provided as means for fixing the cassette housing (1) to the pump, at least one latching lug (4a, 5a) being arranged on each pair (4, 5) of limbs.

9. Tube cassette according to Claim 8, the groove (19) running through the two pairs (4, 5) of limbs and the lateral recess (2) in the cassette housing (1) being delimited on both sides by the two pairs (4, 5) of limbs.

## Revendications

1. Cassette à tube pour pompe péristaltique, avec un boîtier de cassette 1 pourvu d'un creux latéral (2), dans lequel une roue à galets de la pompe péristaltique est destinée à s'engrener, le creux (2) étant au moins partiellement délimité par un élément en forme de segment de cercle (3) qui est au moins partiellement pourvu d'une rainure (19) destinée à recevoir une section de tube (25), le boîtier de cassette (1) étant pourvu de moyens (4, 4a, 5, 5a) pour la fixation sur la pompe, **caractérisé en ce que** la rainure (19) destinée à recevoir la section de tube (25) est prolongée au-delà de la face extérieure (21) opposée à l'élément en forme de segment de cercle du boîtier de cassette (1), de manière que la section de tube (25) à recevoir dans la rainure (19) puisse être introduite et ressortie dans le boîtier de cassette (1) depuis le même côté et **en ce que** sur le boîtier de cassette (1) sont disposés deux bras porteurs (8, 9), qui sont pourvus, côté extrémités, d'une réception (10, 11) servant respectivement à recevoir des stoppeurs (26, 28) fixés sur la section de tube.

2. Cassette à tube suivant la revendication 1, deux faces extérieures (20, 21) au moins du boîtier de cassette (1) étant pourvues d'une section de rainure (19b, 19c) pour former la rainure (19) qui reçoit la section de tube (25).

3. Cassette à tube suivant la revendication 1 ou 2, trois faces extérieures (18, 20, 21) du boîtier de cassette (1) étant pourvues d'une section de rainure (19a, 19b, 19c) pour former la rainure (19) qui reçoit la section de tube (25) et en ce que deux bras porteurs (8, 9) sont disposés sur la quatrième face extérieure (1).

4. Cassette à tube suivant une des revendications précédentes, la réception respective (10, 11) étant pourvue de deux creux (12, 13 ; 15, 16) servant à recevoir respectivement un stoppeur (26, 28) en deux parties.

5. Cassette à tube suivant la revendication 4, entre les deux creux (12, 13 ; 15, 16) étant prévu respectivement un étranglement (14, 17) pour la fixation par blocage du stoppeur respectif (26, 28).

6. Cassette à tube suivant une des revendications précédentes, l'élément en forme de segment de cercle étant conçu comme bras levier qui s'appuie de manière élastique sur le boîtier de cassette (1).

7. Cassette à tube suivant la revendication 6, des moyens (23) de modification de la déviation du bras levier (3) étant prévus.

8. Cassette à tube suivant une des revendications précédentes, deux paires de branches (4, 5) étant prévues comme moyen pour fixer le boîtier de cassette (1) sur la pompe, sur chaque paire de branches (4, 5) étant disposé au moins un tenon d'arrêt (4a, 5a).

9. Cassette à tube suivant la revendication 8, la rainure (19) s'étendant à travers les deux paires de branches (4, 5) et le creux latéral (2) dans le boîtier de cassette (1) étant délimité sur les deux côtés par les deux paires de branches (4, 5).
